(19)

Europäisches
Patentamt
European
Patent Office
Office européen
des brevets

(11) **EP 2 623 980 A1**

(12) **EUROPEAN PATENT APPLICATION**
published in accordance with Art. 153(4) EPC

(43) Date of publication:
**07.08.2013 Bulletin 2013/32**

(21) Application number: **11828424.9**

(22) Date of filing: **28.09.2011**

(51) Int Cl.:
***G01N 33/543*** (2006.01)　　　***G01N 21/78*** (2006.01)

(86) International application number:
**PCT/JP2011/005440**

(87) International publication number:
**WO 2012/042858 (05.04.2012 Gazette 2012/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.09.2010 JP 2010218428**

(71) Applicant: **FUJIFILM Corporation
Tokyo 106-8620 (JP)**

(72) Inventor: **ARAI, Hisao
Ashigarakami-gun
Kanagawa 258-8538 (JP)**

(74) Representative: **HOFFMANN EITLE
Patent- und Rechtsanwälte
Arabellastrasse 4
81925 München (DE)**

(54) **IMMUNOCHROMATOGRAPHIC INSPECTION METHOD AND DEVICE**

(57)　　[Aim] To obtain an immunochromatographic assay method that can accurately measure a color-developed state by a reagent.

[Solution Means] In an immunochromatographic assay method for performing an assay related to a specimen by reacting a reagent held by a carrier and the specimen with each other, and by obtaining an image signal by imaging a reagent portion by a camera (42), and by measuring, based on the image signal, the color-developed state of the reagent, a morphological opening operation is applied to the image signal by using a structuring element the size of which is less than or equal to a signal change width of the reagent portion that has developed color, and a morphological opening operation is applied to the image signal by using a structuring element the size of which is larger than the signal change width of the reagent portion that has developed color. The color-developed state is measured based on a difference signal obtained by performing a subtraction between the image signals to which the opening operations have been applied respectively.

FIG.10

EP 2 623 980 A1

**Description**

BACKGROUND OF THE INTENTION

Field of the Invention

The present invention relates to an assay method and apparatus using immunochromatography.

Description of the Related Art

In recent years, many devices for assay that can easily and quickly test an analyte by using an assay method, such as immunoassay, were developed. In such devices for assay, a specimen (sample) that may contain an analyte is held by a carrier, and assayed. Further, various devices for testing in-vitro diagnostic reagents, toxic substances or the like are commercially available. Some example of such devices uses immunochromatography, as disclosed in Japanese Unexamined Patent Publication No. 2008-139297 (Patent Document 1). When a device adopting immunochromatography is used, an assay result is obtainable by leaving a specimen solution held by a carrier for only about 5 to 10 minutes at the shortest. Therefore, assay techniques using assay methods, such as immunoassay, are widely adopted as simple and quick assay techniques, for example, in a clinical test at a hospital, an assay test at a laboratory, or the like.

Especially, in medical care situations at a doctor's office, a clinic, home and the like, many immunochromatographic assay apparatuses (immunochromato-readers) are used as assay devices for POCT (Point of Care Testing) care. In POCT, a simple assay is possible without relying on a clinical test specialist. The immunochromatographic assay apparatus can measure the color-developed state of a reagent in a device loaded into the apparatus at high sensitivity. Therefore, even if the color-developed state is too low to visually judge the state, a highly sensitive and reliable assay is possible. Japanese Unexamined Patent Publication No. 2009-133813 (Patent Document 2) discloses an example of this kind of assay apparatus.

In the aforementioned assay methods, it is necessary to detect an extremely small amount of analyte at high sensitivity. As an assay method satisfying such a need, a method that performs amplification (sensitization), for example, as disclosed in Japanese Unexamined Patent Publication No. 2009-287952 (Patent Document 3) is known. In this method, after an analyte is developed on a carrier, washing liquid is supplied to wash away everything except a labeling substance captured by binding to a specific reaction site on the carrier. After then, a sensitizing solution is supplied onto the carrier to sensitize the labeling substance. Accordingly, a very small amount of analyte becomes detectable at high sensitivity.

Here, the aforementioned sensitizing process may be performed only if necessary. In other words, if the color-developed state of a reagent has been measured by ordinary processing, measurement may end. If the color-developed state is not measurable by ordinary processing, the sensitizing process may be performed, and the color-developed state may be measured after the sensitizing process.

SUMMARY OF THE INVENTION

In many immunochromatographic assay apparatuses as described above, an image signal is obtained by imaging a reagent portion that has been reacted with a specimen, and the color-developed state of the reagent reacted with the specimen is measured based on the image signal. However, in such cases, the image signal contains a large noise component caused by the granular characteristic of the reagent portion or the like. Therefore, it is difficult to accurately measure the color-developed state in some cases.

In view of the foregoing circumstances, it is an object of the present invention to provide an immunochromatographic assay method and apparatus that can accurately measure the color-developed state by eliminating the influence of such noise.

As described above, an immunochromatographic assay method of the present invention is an immunochromatographic assay method for performing an assay related to a specimen by reacting a reagent held by a carrier and the specimen with each other, and by obtaining an image signal by imaging a reagent portion that has been reacted, and by measuring, based on the image signal, the color-developed state of the reagent caused by the reaction,
the method comprising the steps of:

applying a morphological opening operation to the image signal by using a structuring element the size of which is less than or equal to a signal change width of the reagent portion that has developed color;
applying a morphological opening operation to the image signal by using a structuring element the size of which is larger than the signal change width of the reagent portion that has developed color;

obtaining a difference signal by performing a subtraction between the image signals to which the opening operations have been applied respectively; and

measuring the color-developed state based on the difference signal.

Further, another immunochromatographic assay method of the present invention is an immunochromatographic assay method for performing an assay related to a specimen by reacting a reagent held by a carrier and the specimen with each other, and by obtaining an image signal by imaging a reagent portion that has been reacted, and by measuring, based on the image signal, the color-developed state of the reagent by the reaction,

the method comprising the steps of:

applying a level inversion operation to the image signal;

applying a morphological closing operation to the image signal after the level inversion operation by using a structuring element the size of which is less than or equal to a signal change width of the reagent portion that has developed color;

applying a morphological closing operation to the image signal after the level inversion operation by using a structuring element the size of which is larger than the signal change width of the reagent portion that has developed color;

obtaining a difference signal by performing a subtraction between the image signals to which the closing operations have been applied respectively; and

measuring the color-developed state based on the difference signal.

Here, the term "signal change width" does not mean a change width of an image signal on a time axis, but a distance between two ends of a reagent portion that has changed an image signal obtained by imaging the color-developed reagent portion.

In the immunochromatographic assay method of the present invention, it is desirable that the reagent held by the carrier has a band shape with a predetermined width.

In the immunochromatographic assay method of the present invention, it is desirable that the shape of the structuring element the size of which is less than or equal to the signal change width is more rectangular as the intensity of the image signal is higher, and more arc-shaped as the intensity of the image signal is lower.

The shape of the structuring element the size of which is less than or equal to the signal change width may be bilaterally asymmetrical.

If the structuring element the size of which is less than or equal to the signal change width is too small, an influence of noise tends to increase. Therefore, it is desirable that the size of the structuring element is as close to the signal change width as possible. In practical application, it is desirable that the size of the structuring element is 60% or greater of the signal change width. It is more desirable to use the structuring element the size of which is 80% or greater of the signal change width.

In contrast, it is desirable in practical application that the size of the structuring element the size of which is larger than the signal change width is 1.2 times or more as large as the signal change width. It is more desirable to use the structuring element the size of which is 1.5 times or more as large as the signal change width.

Here, the desirable size of the structuring element the size of which is less than or equal to the signal change width, and the desirable size of the structuring element the size of which is larger than the signal change width may be changed appropriately based on the width of the reagent portion. For example, when the width of the reagent portion is 1 mm, it is desirable that the size of the former structuring element is substantially in the range of 0.1 to 1 mm, and that the size of the latter structuring element is substantially larger than 1 mm and less than or equal to 1.5 mm.

Meanwhile, an immunochromatographic assay apparatus of the present invention is an immunochromatographic assay apparatus comprising:

a loading means that loads a carrier holding a reagent;

an imaging means that obtains an image signal by imaging a reagent portion that has been reacted with a specimen; and

a measuring means that measures, based on the image signal, the color-developed state of the reagent by the reaction,

the apparatus comprising:

a morphological operation unit that applies a morphological opening operation to the image signal by using a structuring element the size of which is less than or equal to a signal change width of the reagent portion that has developed color, and that applies a morphological opening operation to the image signal by using a structuring element the size of which is larger than the signal change width of the reagent portion that has developed color;

a subtraction processing unit that obtains a difference signal by performing a subtraction between the image signals to which the opening operations have been applied respectively; and
a measuring means that measures the color-developed state based on the difference signal.

Another immunochromatographic assay apparatus of the present invention is an immunochromatographic assay apparatus comprising:

a loading means that loads a carrier holding a reagent;
an imaging means that obtains an image signal by imaging a reagent portion that has been reacted with a specimen; and
a measuring means that measures, based on the image signal, the color-developed state of the reagent by the reaction,
the apparatus comprising:

a means for applying a level inversion operation to the image signal;
a morphological operation unit that applies a morphological closing operation to the image signal by using a structuring element the size of which is less than or equal to a signal change width of the reagent portion that has developed color, and that applies a morphological closing operation to the image signal by using a structuring element the size of which is larger than the signal change width of the reagent portion that has developed color;
a subtraction processing unit that obtains a difference signal by performing a subtraction between the image signals to which the closing operations have been applied respectively; and
a measuring means that measures the color-developed state based on the difference signal.

First, morphological operations adopted in this invention will be described. A morphological (Morphology) operation is referred to as morphology, or morphology operation. In general, the morphological operation is developed as a set theory in N-dimensional space, but often applied to an image of two-dimensional space (please refer to Japanese Unexamined Patent Publication No. 8(1996) -272961, Japanese Unexamined Patent Publication No. 9(1997)-248291, Japanese Unexamined Patent Publication No. 9(1997)-091421, and the like). Next, the morphological operation will be briefly described with respect to a case in which a grayscale image is a processing target as in the present invention.

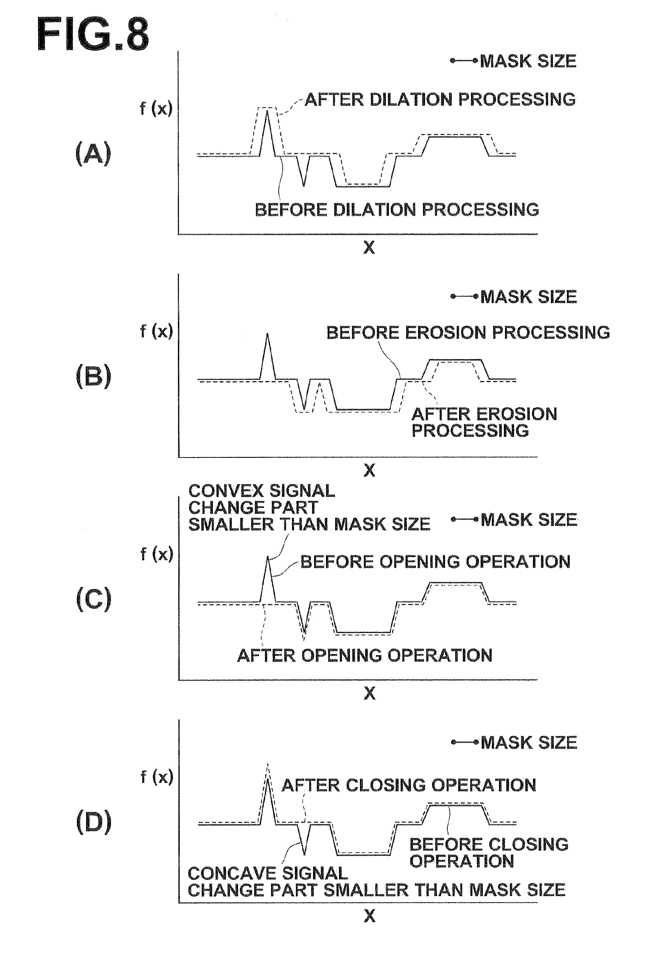

A grayscale image is regarded as a space in which a point at coordinate $(x, y)$ has a height corresponding to grayscale value $f(x, y)$. As illustrated in Figure 8, one-dimensional function $f(x)$ corresponding to a cross-section of this space is considered. Structuring element $g$ used in the morphological operation is a symmetric function with respect to an origin, as shown in the following formula (1):

[Formula 1]

$$g^s(x) = g(-x) \quad \dots (1).$$

The value is 0 in the domain of the function, and domain G of the function is represented by the following formula (2):

[Formula 2]

$$G = \{-m, -m-1, \cdots, -1, 0, 1, \cdots, m-1, m\} \quad \dots (2).$$

At this time, basic morphological operations are extremely simple, as represented by the following formulas (3) through (6):

[Formula 3]

$$\text{dilation;} \left[ f \oplus G^s \right](i) = \max\{f(i-m), \cdots, f(i), \cdots, f(i+m)\} \quad \dots (3);$$

$$\text{erosion;} \left[ f \otimes G^s \right](i) = \min\{f(i-m), \cdots, f(i), \cdots, f(i+m)\} \quad \dots (4);$$

$$\text{opening;} f_g = \left( f \otimes g^s \right) \oplus g \quad \dots (5);$$

and

$$\text{closing; } f_g = \left(f \oplus g^s\right) \otimes g \qquad \ldots (6).$$

Specifically, a dilation (dilation) operation searches a range with a width of $\pm m$ (a value determined based on structuring element B, and the value corresponds to a mask size in Figure 8) with respect to a pixel of interest, as a center, for a maximum value (please refer to Figure 8(A)). In contrast, an erosion (erosion) operation searches a range with a width of $\pm m$ with respect to a pixel of interest, as a center, for a minimum value (please refer to Figure 8(B)).

Further, in an opening (opening) operation, a dilation operation is performed after an erosion operation. In other words, search for a maximum value is performed after search for a minimum value. In contrast, in a closing (closing) operation, an erosion operation is performed after a dilation operation. In other words, search for a minimum value is performed after search for a maximum value. Specifically, the opening operation smoothes density curve f(x) from the low density side of the curve, and corresponds to suppression of a convex density fluctuation part (a part in which the density is higher than a surrounding part thereof) that fluctuates in a spatially narrower range than mask size 2m (please refer to Figure 8(C)). In contrast, the closing operation smoothes density curve f(x) from the high density side of the curve, and corresponds to suppression of a concave density fluctuation part (a part in which the density is lower than a surrounding part thereof) that fluctuates in a spatially narrower range than mask size 2m (please refer to Figure 8(D)).

Here, when signals are high-density high-signal-level signals, the values of which are higher as the densities are higher, the relationship of the numerical order of image signal values representing density values f(x) is opposite to that of the case of high-luminance high-signal-level signals. Therefore, a dilation operation on the high-density high-signal-level signals is the same as an erosion operation on the high-luminance high-signal-level signals (Figure 8(B)). Further, an erosion operation on the high-density high-signal-level signals is the same as a dilation operation on the high-luminance high-signal-level signals (Figure 8(A)). Further, an opening operation on the high-density high-signal-level signals is the same as a closing operation on the high-luminance high-signal-level signals (Figure 8(D)). Further, a closing operation on the high-density high-signal-level signals is the same as an opening operation on the high-luminance high-signal-level signals (Figure 8(C)).

Therefore, it is possible to suppress the granularity of the image (representing noise, as image signals), or to remove the granularity from the image by applying a morphological opening operation or a morphological closing operation to image signals representing an original image, as described above (please refer to "Morphology", Obata, CORONA PUBLISHING CO., LTD., and the like).

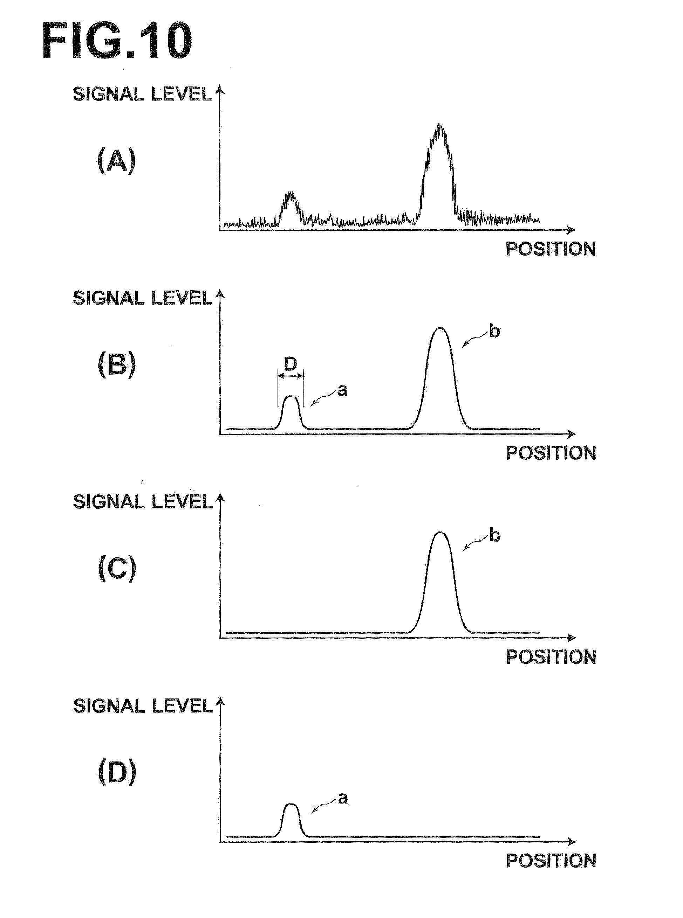

In the immunochromatographic assay method of the present invention, a morphological opening operation is applied to an image signal representing a reagent portion that has been reacted with a specimen by using a structuring element the size of which is less than or equal to a signal change width of the reagent portion that has developed color, and a morphological opening operation is applied to the image signal by using a structuring element the size of which is larger than the signal change width of the reagent portion that has developed color. Therefore, for example, when an original image signal is as illustrated in Figure 10(A), the image signal after the former opening operation is smoothed as illustrated in Figure 10 (B). Meanwhile, the image signal after the latter opening operation is smoothed as illustrated in Figure 10(C) (in these diagrams, a represents a signal component representing a reagent portion, and b represents a signal component representing a portion, such as a control line, which is not directly related to an assay of a specimen, which will be described later). In other words, since the structuring element the size of which is less than or equal to signal change width D of the reagent portion is used in the former opening operation, the signal component a remains after the opening operation. However, since a structuring element the size of which is larger than the signal change width D is used in the latter opening operation, the signal component a is eliminated after the opening operation.

Therefore, when a subtraction operation is performed between the image signals to which the two opening operations have been applied respectively, a difference signal as illustrated in Figure 10(D) is obtainable. Specifically, the signal component a representing the reagent portion clearly remains. Therefore, if this difference signal is used, even if the signal component a that has changed by the color development of the reagent portion is very weak, it is possible to eliminate the influence of noise contained in the original image signals, and to accurately measure the color-developed state of the reagent portion.

Here, the signal component a representing the reagent portion is more rectangular as the intensity of the original image signal is higher, and more arc-shaped as the intensity of the original image signal is lower. Therefore, in the immunochromatographic assay method of the present invention, especially when a structuring element that is more rectangular as the intensity of the image signal is higher and more arc-shaped as the intensity of the image signal is lower is used as a structuring element the size of which is less than or equal to the signal change width, image signals after an opening operation using the structuring element more clearly maintains a change in the image signals by color development. Further, in this case, the difference signal more clearly maintains a change caused by color development.

Therefore, the accuracy in measurement of the color-developed state becomes even higher.

Especially when the intensity of the original image signal is low, the shape of the signal component a representing the reagent portion tends to be bilaterally asymmetrical. Therefore, when the original image signal is bilaterally asymmetrical, if a bilaterally asymmetrical structuring element that matches with the asymmetrical shape of the original image signal is used as a structuring element the size of which is less than or equal to a signal change width, it is possible to more certainly eliminate the influence of noise. The bilateral symmetry characteristic of the original image signals tends to be unique to each assay apparatus in many cases. Therefore, if the tendency of the asymmetry characteristic is studied in advance, it is possible to match the bilateral asymmetry characteristic of the structuring element and the bilateral asymmetry characteristic of the image signal with each other.

Further, the intensity of the image signal tends to become low when the amount of applied reagent is small. Generally, when the application amount is small, it is difficult to uniformly apply the reagent. Therefore, when the application amount is small, it is desirable to apply the reagent in such a manner that the application amount of the reagent has predetermined distribution, instead of uniform application, thereby a predetermined tendency of bilateral asymmetry characteristic is given to the image signal. Then, it is possible to set the bilateral asymmetry characteristic of the structuring element so as to closely match with the bilateral asymmetry characteristic of the image signal by obtaining the tendency of bilateral asymmetry characteristic in advance.

If it is possible to accurately measure the color-developed state of the reagent portion as described above, an assay of a specimen based on the measurement becomes highly accurate and reliable.

Meanwhile, an immunochromatographic assay apparatus of the present invention includes a loading means that loads a carrier holding a reagent, an imaging means, a measuring means that measures, based on an image signal, the color-developed state of the reagent, a morphological operation unit, a subtraction processing unit that obtains a difference signal, and a measuring means that measures the color-developed state based on the difference signal, as described above. Therefore, this apparatus can perform the immunochromatographic assay method of the present invention.

BRIEF DESCRIPTION OF THE DRAWINGS

Figure 1 is a schematic diagram illustrating an immunochromatographic assay apparatus according to an embodiment of the present invention;
Figure 2 is a partially-broken side view of the assay apparatus;
Figure 3 is a block diagram illustrating the electrical configuration of the assay apparatus;
Figure 4 is a front view illustrating a part of the assay apparatus;
Figure 5 is a partially-broken plan view illustrating a state of a cartridge used in the assay apparatus;
Figure 6 is a partially-broken plan view illustrating another state of the cartridge;
Figure 7 is a partially-broken plan view illustrating still another state of the cartridge;
Figure 8 is an explanatory diagram for explaining morphological operations;
Figure 9 is a flow chart illustrating the flow of assay processing by the assay apparatus; and
Figure 10 is an explanatory diagram for explaining an effect of the present invention.

DESCRIPTION OF THE PREFERRED EMBODIMENTS

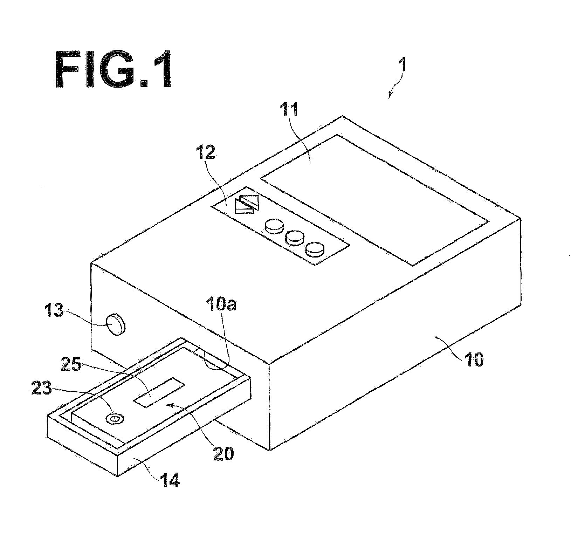

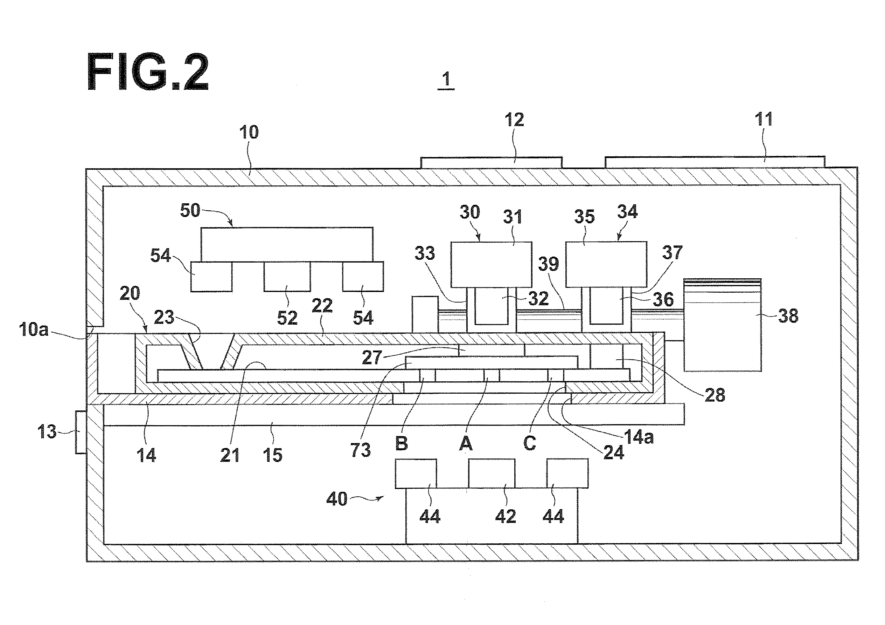

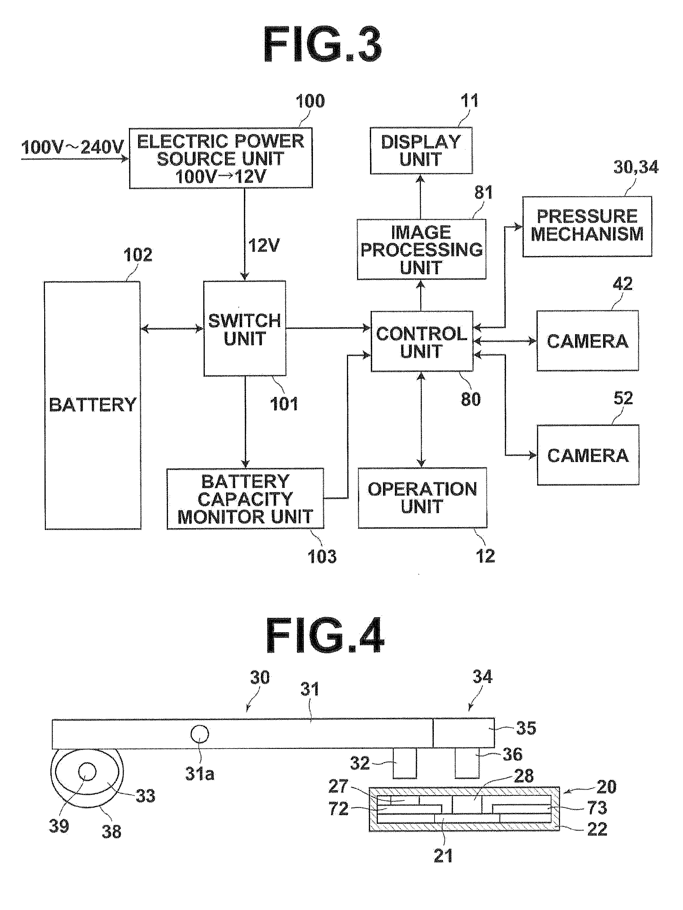

Hereinafter, embodiments of the present invention will be described in detail with reference to drawings. Figure 1 is a perspective view of an immunochromatographic assay apparatus 1 according to an embodiment of the present invention. Figure 2 is a partially-broken side view of the immunochromatographic assay apparatus 1. Figure 3 is a diagram illustrating the electrical configuration of the immunochromatographic assay apparatus 1. First, the basic structure of the immunochromatographic assay apparatus 1 of the present invention will be described with reference to Figures 1 and 2.

As illustrated in these diagrams, the immunochromatographic assay apparatus 1 includes a case 10 having an opening 10a on the front side thereof, a display unit 11 arranged on the upper surface of the case 10, an operation unit 12 for operating a menu displayed on the display unit 11, an electric power switch 13, and a cartridge loading unit 14 for loading a cartridge 20 for immunochromatography into the apparatus. Further, the immunochromatographic assay apparatus 1 has a rail 15 that guides the cartridge loading unit 14 in such a manner that the cartridge loading unit 14 is movable in the horizontal direction in Figure 2, pressure units 30, 34 for squeezing a washing liquid pot 27 and a sensitizing solution pot 28, which will be described later, respectively, and a first measurement unit 40 and a second measurement unit 50 for obtaining information from the cartridge 20 in the case 10.

The cartridge loading unit 14 is automatically or manually movable along the rail 15. When a most part of the cartridge loading unit 14 has moved to the outside of the case 10 through the opening 10a, a cartridge 20, into which assay solution was supplied as will be described later, is loaded on the cartridge loading unit 14. After then, the cartridge loading unit 14 is pushed into the case 10, as illustrated in Figure 2. Accordingly, the cartridge 20 is loaded into the immunochromatographic assay apparatus 1.

Figure 4 is a front view of the pressure units 30, 34, viewed from the left side of Figure 2. Here, a broken view of the cartridge 20 is illustrated. With reference to Figure 4, the pressure units 30, 34 will be described. The pressure unit 30 includes an arm 31 that is pivotable like a seesaw around a shaft 31a, a pressure portion 32 fixed onto a lower surface of a leading end of the arm 31, and a cam 33 arranged on the lower side of a rear end of the arm 31. The cam 33 is connected to a drive shaft 39 that is rotated by a motor 38, for example, in such a manner that the cam 33 is disconnectable from the drive shaft 39 through an electromagnetic clutch or the like, which is not illustrated. When the cam 33 rotates, the rear end of the arm 31 is moved up, and the pressure portion 32 at the leading end is moved down. Further, the other pressure unit 34 includes an arm 35, a pressure portion 36, and a cam 37, and is structured in a similar manner to the pressure unit 30.

Here, the pressure portion 32 of the pressure unit 30 and the pressure portion 36 of the pressure unit 34 are arranged in such a manner that the pressure portion 32 and the pressure portion 34 are located just above the washing liquid pot 27 and the sensitizing solution pot 28 arranged in the kit, respectively, when the cartridge 20 is placed at a predetermined position in the case 10.

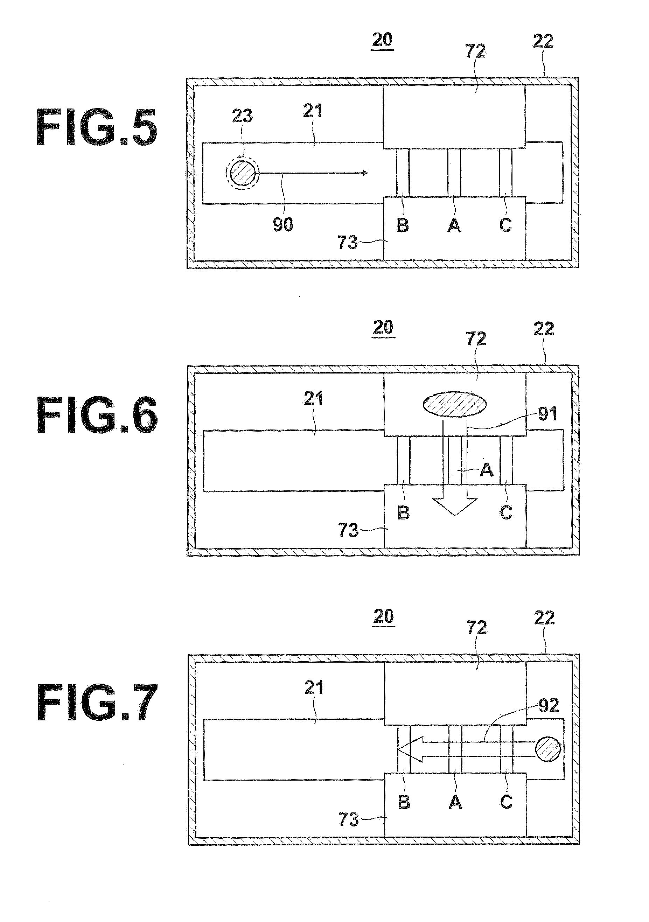

Figure 5 is a plan view illustrating the cartridge 20, removing the upper surface of the cartridge 20. The cartridge 20 will be described with reference to Figure 5. The cartridge 20 includes an insoluble carrier 21, a kit case 22 for housing the insoluble carrier 21, a solution injection hole 23, and an observation window 24. The insoluble carrier 21 includes test line A that carries a reagent in a band shape, test line B that carries a reagent in a band shape, and band-shaped control line C. The solution injection hole 23 is formed on the upper surface of the case to inject specimen solution onto the soluble carrier 21. The observation window 24 is provided to observe assay sites (test lines A, B and control line C portions) of the insoluble carrier 21. An information display unit 25 is provided on the upper surface of the kit case 22. Further, an observation window 14a that substantially matches with the observation window 24 is provided on the cartridge loading unit 14.

The insoluble carrier 21 includes an immobilized labeling substance. Further, each of the test lines A and B is formed by a substance that specifically binds to the specimen (analyte), as a reagent. The control line C is provided to judge completion of measurement.

Further, an insoluble carrier 72 for supplying liquid and an insoluble carrier 73 for absorption are arranged on both sides of the insoluble carrier 21 in the cartridge 20. Further, the aforementioned washing liquid pot 27 is fixed at a position above the insoluble carrier 72 for supplying liquid, and the sensitizing solution pot 28 is fixed at a position above a control line C side end of the insoluble carrier 21. Further, the upper surface of the case 22 of the cartridge 20 is easily deformable when the case 22 is pressed down by the aforementioned pressure portions 32, 36, and allows the washing liquid pot 27 and the sensitizing solution pot 28 to be squeezed by the pressure portions 32, 36, respectively.

The first measurement unit 40 measures the color-developed state at assay sites (test line A, B and control line C portions) through the observation window 24 of the cartridge 20. As illustrated in Figure 2, the first measurement unit 40 includes a camera 42 and a light source 44. The first measurement unit 40 is structured in such a manner that the camera 42 and the light source 44 face the observation window 24 from the lower side of the cartridge 20 when the cartridge 20 is loaded into the assay apparatus 1. Further, optical density and chromaticity are calculated, as the color-developed state of the assay sites, based on the optical information about the assay sites obtained by the first measurement unit 40 (this will be described later).

Here, when the intensity of incident light entering an assay site in the cartridge 20 is I, and the intensity of reflection light from the assay site is $I_r$, the optical density is defined by the following formula:

$$\texttt{Optical Density} = -\log_{10}(\texttt{I}_r/\texttt{I}).$$

Further, chromaticity numerically represents hue and saturation. The chromaticity is calculated based on an RGB luminance signal read by the camera. A general CIE color system may be used as the color system of chromaticity.

The camera 42 includes an image sensor, for example, in which plural photodiodes are linearly arranged, or an area sensor. The camera 42 outputs a signal based on the light amount of received light. The light receiving range of the camera 42 is a band-shaped range extending along the longitudinal direction of the cartridge 20. The light source 44 is, for example, a module in which an LED is incorporated, and structured so as to output white light. The light source 44 may output, for example, light of a single color as long as a change in chromaticity by sensitizing process, which will be described later, is distinguishable. When the light source 44 is composed of plural modules, it maybe composed of plural modules, each outputting light of a single color the wavelength of which is different from each other. Light output from the light source 44 can illuminate a predetermined range in the longitudinal direction of the cartridge 20.

Meanwhile, the second measurement unit 50 obtains information displayed on the information display unit 25 of the cartridge 20 by illuminating the information display unit 25 with illumination light. The information display unit 25 displays information about an assay by hand writing, by adhesion of a sticker, or the like. The information about an assay is, for example, information about a patient from whom an analyte has been collected (name, age, sex, and the like), information about a sample and a reagent used in the assay (a specimen that is a target of assay, the names of washing liquid, sensitizing solution, and the like), and the like. The method for obtaining the information is not particularly limited. What is displayed on the information display unit 25 may be directly imaged, or bar-coded information may be read.

As illustrated in Figure 2, the second measurement unit 50 includes a camera 52 and a light source 54. The second measurement unit 50 is structured in such a manner that the camera 52 and the light source 54 face the information display unit 25 from the upper side of the cartridge 20 when the cartridge 20 is loaded into the assay apparatus 1. Further, the information about the assay obtained by the second measurement unit 50 and an assay result are managed in such a manner that they are linked with each other. Specific structures of the camera 52 and the light source 54 are similar to those of the camera 42 and the light source 44, which were described already, respectively.

Next, the electrical configuration of the apparatus of the present invention will be described with reference to Figure 3. Operations of the display unit 11, the operation unit 12, the pressure mechanisms 30, 34 including the motor 38 and the like, and the cameras 42, 52 (including the light sources 44, 54, respectively), which were described already, are controlled by a control unit 80 illustrated in Figure 3. Further, the assay apparatus 1 of the present invention can operate, for example, by using a commercial electric power source of 100 through 240V. The assay apparatus 1 includes an electric power source unit 100 for converting electricity received from the commercial electric power source into direct current of 12V, and a switch unit 101 to which the direct current of 12V is input. Further, the assay apparatus 1 of the present invention can be driven by a battery 102, which is a secondary battery. The battery 102 is also connected to the switch unit 101. The switch unit 101 performs switching in such a manner that direct current of 12V supplied from the electric power source unit 100 is used by each electrical part when the switch unit 101 is connected to the commercial electric power source, and that direct current of 12V supplied from the battery 102 is used by each electrical part when the switch unit 101 is not connected to the commercial electric power source.

Further, a battery capacity monitor unit 103 is connected to the switch unit 101. The battery capacity monitor unit 103 is a battery remaining-amount detection means for detecting the remaining electric power amount of the battery 102. Generally, because of the chemical properties of a battery, the internal resistance of the battery increases as the capacity of the battery becomes lower, and terminal voltage of the battery becomes lower. Therefore, it is possible to detect the remaining electric power amount of the battery by measuring the terminal voltage of the battery. The battery capacity monitor unit 103 continues detection of the remaining electric power amount of the battery 102 in this manner, and inputs a signal representing the remaining electric power amount to the control unit 80.

Next, measurement by the assay apparatus 1 in the present embodiment will be described. In principle, the apparatus of the present invention performs first stage measurement and second stage measurement, which follows the first stage measurement. The first stage measurement measures the color-developed state of the assay sites without sensitizing process, which will be described later. The second stage measurement measures the color-developed state of the assay sites after sensitizing process, which will be described later.

Next, specific measurement steps will be described.

«First Stage Measurement»

In the first stage measurement, first, specimen solution 90 is injected through the solution injection hole 23 of the cartridge 20 located on the outside of the assay apparatus 1, for example, as illustrated in Figure 5. Then, the cartridge 20 is placed in the assay apparatus 1, as described already, and images of assay sites (test line A, B and control line C portions) in the cartridge 20 are imaged by the camera 42 to calculate the optical density and the chromaticity of the assay sites. The control unit 80 illustrated in Figure 3 inputs a two-dimensional image signal output by the camera 42 to the image processing unit 81.

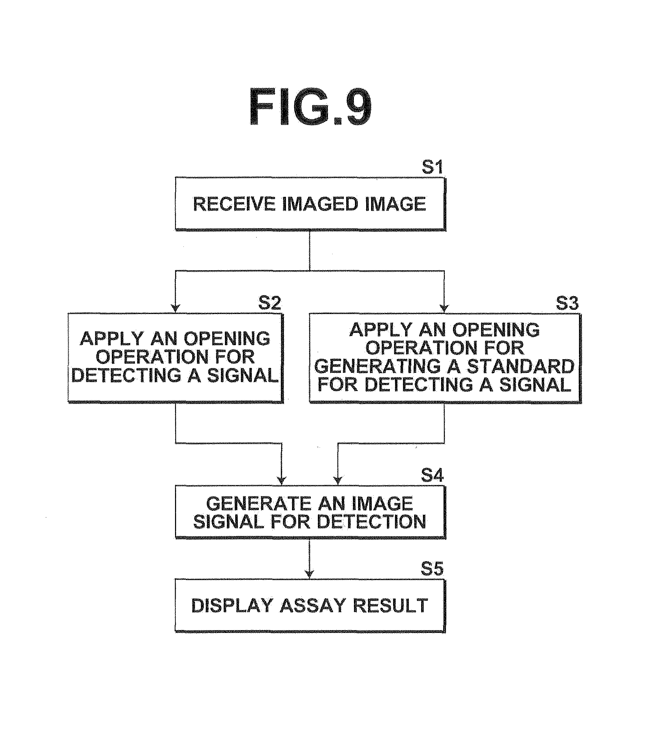

Next, the flow of image processing by the image processing unit 81 and the flow of displaying an assay result, following the image processing, will be described also with reference to Figure 9. First, an image signal representing the two-dimensional image is input to the image processing unit 81 (step S1 in Figure 9, and so on). Next, the image processing unit 81 performs two opening operations (steps S2 and S3) illustrated in Figure 9. However, for that purpose, the image processing unit 81 generates a one-dimensional image signal from the two-dimensional image signal.

The one-dimensional image signal represents one-dimensional density distribution on the soluble carrier 21 along a straight line crossing lines A, B and C, illustrated in Figures 5 through 7. The one-dimensional image signal is generated, for example, by extracting image signals with respect to many points along a straight line that passes the vicinity of the central position of each of the lines A, B and C from the two-dimensional image signal, or by extracting image signals with respect to many points along the straight line and a few straight lines that are parallel to the straight line, and by adding the image signals the positions of which are the same in the arrangement direction of the lines A, B and C (the horizontal direction in Figures 5 through 7) together, or further by obtaining an average of the signals after the addition.

After then, the image processing unit 81 performs a morphological opening operation for detecting a signal (step S2) and a morphological opening operation for generating a standard for detecting a signal (step S3) on the one-dimensional image signal. The former opening operation is performed by using a structuring element the size of which is less than or equal to signal change width D of the color-developed reagent portion, which was described already with reference to Figure 10. The latter opening operation is performed by using a structuring element the size of which is larger than the signal change width D of the color-developed reagent portion, which was described already also with reference to Figure 10.

The image processing unit 81 first stores each of one-dimensional image signals to which the two opening operations have been applied respectively in an internal memory. Then, the image processing unit 81 performs processing for generating an image signal for detecting whether a disease is present or not, or the like on the image signals (step S4). The processing for generating an image signal for detection is a subtraction operation, which was described already with reference to Figure 10. Accordingly, a difference signal as illustrated in Figure 10(D) is obtained. When the difference signal is used, it is possible to accurately measure the color-developed state of the reagent portion, excluding the influence of noise included in the original two-dimensional image signal as described already. The details are as described with reference to Figure 10 already.

The image processing unit 81 calculates the color-developed state of the reagent portion, in other words, the optical density and chromaticity based on the difference signal. Further, the image processing unit 81 displays these values, or the presence or non-presence of a disease judged based on the values, or the like on the display unit 11, as a detection result (step S5). If the color-developed state of the reagent portion is accurately measurable as described above, the detection result is highly reliable.

As clearly described, the image processing unit 81 constitutes a morphological operation unit, a subtraction processing unit for obtaining a difference signal, and a means for measuring the color-developed state in the present embodiment.

Further, it is desirable that the shape of the structuring element used in the opening operation for detecting a signal (step S2) is based on the shape of the reagent portion. In contrast, it is desirable that the shape of the structuring element used in the opening operation for generating a standard for detecting a signal (step S3) is always constant, because the structuring element is a base of the difference signal, in other words, a difference between the intensities of signals in the processing for generating a standard.

«Second Stage Measurement»

In the second stage measurement, first, the pressure unit 30 illustrated in Figures 2 and 4 is driven, and the leading end of the arm 31 of the pressure unit 30 moves down, and the pressure portion 32 squeezes the washing liquid pot 27 in the cartridge 20 from the outside of the cartridge 20. Accordingly, as illustrated in Figure 6, washing liquid 91 stored in the washing liquid pot 27 washes the assay site of the insoluble carrier 21. At this time, the washing liquid 91 is supplied to the insoluble carrier 21 and the insoluble carrier 73 for absorption in this order after the washing liquid 91 has sufficiently spread on the insoluble carrier 72 for supplying liquid.

Next, the pressure unit 34 illustrated in Figures 2, 4 is driven, and the leading end of the arm 35 of the pressure unit 34 moves down, and the pressure portion 36 squeezes the sensitizing solution pot 28 in the cartridge 20 from the outside of the cartridge 20. Accordingly, as illustrated in Figure 7, sensitizing solution 92 stored in the sensitizing solution pot 28 is supplied to the assay site of the insoluble carrier 21, and sensitization is performed. The details of the sensitizing solution 92 and the washing liquid 91 are disclosed in detail in Patent Document 3, and the disclosure of Patent Document 3 is adoptable also in the present invention.

After the sensitizing process, an image of the assay site in the cartridge 20 is imaged by the camera 42 in a manner similar to the aforementioned case. The control unit 80 illustrated in Figure 3 inputs a two-dimensional image signal output by the camera 42 to the image processing unit 81. The image processing unit 81 performs the same processing as the processing performed in the first stage measurement on this two-dimensional image signal. Accordingly, it is possible to accurately measure the color-developed state of the reagent portion based on the finally obtained difference signal.

The image processing unit 81 calculates the color-developed state of the reagent portion, in other words, the optical density and chromaticity of the region based on the difference signal. Further, the image processing unit 81 displays these values, or the presence or non-presence of a disease judged based on the values, or the like on the display unit 11, as a detection result. Since the color-developed state of the reagent portion is accurately measurable also in this case, the detection result is highly reliable.

In the embodiment as described above, a one-dimensional pixel signal is generated based on a two-dimensional image signal, and a one-dimensional morphological operation is applied to the one-dimensional pixel signal. Alternatively, a two-dimensional morphological operation may be applied to a two-dimensional image signal, and a one-dimensional image signal may be generated based on the two-dimensional image signal after the operation.

In the aforementioned embodiment, a morphological opening operation was applied to the image signal obtained by imaging the reagent portion. Since an opening operation applied to a high-density high-signal-level signal and a closing operation applied to a high-luminance high-signal-level signal are the same, the same action and effect as those of the aforementioned embodiment are achievable by performing level inversion processing on the image signal obtained by imaging the reagent portion, and by applying a morphological closing operation to the image signal after the inversion processing. For that purpose, a function for performing level inversion processing on an image signal and a function for applying a closing operation should be provided in the image processing unit 81, illustrated in Figure 3.

Here, matters related to the aforementioned measurement will be briefly described.

(Specimen solution)

Specimen solution that can be assayed by using the assay apparatus of the present invention is not particularly limited as long as the specimen solution may contain an analyte (a physiologically active substance, such as natural products, a toxin, a hormone or an agricultural chemical, an environmental pollutant, and the like). Examples of the specimen solution are a biological sample, in particular, body fluids (for example, blood, blood serum, blood plasma, spinal fluid, lacrimal fluid, sweat, urine, pus, nasal mucus or sputum) or excrements (for example, feces), organs, tissues, mucosae and skin of an animal (particularly, a human), a swab specimen (swab), and a gargle specimen that may contain such a substance, animals or plants themselves or dried bodies thereof diluted with a diluting agent that will be described later, and the like.

The specimen solution may be used directly, or in the form of extracted solution obtainable by using a solvent for extraction appropriate for the specimen solution, or in the form of diluted solution obtainable by further diluting the extracted solution with an appropriate diluting agent, or in the form of condensed solution obtainable by condensing the extracted solution using an appropriate method.

(Labeling substance)

A labeling substance usable in the present invention is not particularly limited as long as the labeling substance has a color and is visually recognizable, or the labeling substance becomes testable by reaction. For example, the labeling substance may be metal particles (or metal colloid), colored latex particles, enzymes, or the like, which are used in general immunochromatography. When signals are amplified by deposition of metal on a labeling substance by a reduction reaction of metal ions with the labeling substance acting as a catalyst, it is desirable to use metal particles from the view point of the catalystic activity thereof.

As the material of the metal particles, a simple metal, a metal sulfide, a metal alloy, or a polymer particle label containing metal may be used. It is desirable that the average particle diameter of the particles (or colloid) is in the range of 1 nm to 10 $\mu$m. Here, the average particle diameter is an average value of diameters (the largest diameter of each particle) of plural particles actually measured by a transmission electron microscope (TEM). Specifically, gold colloid, silver colloid, platinum colloid, iron colloid, aluminum hydroxide colloid, composite colloid thereof, and the like may be used. It is desirable to use gold colloid, silver colloid, platinum colloid, and composite colloid thereof. Especially, gold colloid, silver colloid and composite colloid thereof are desirable. They are desirable because the gold colloid shows red, and the silver colloid shows yellow when the particle diameters are appropriate, and they are easily visually recognizable. When the gold colloid is used, the chromaticity of the label changes after a sensitizing process using a silver ion containing compound (the gold colloid shows red, and after the sensitizing process, the red color changes to black by deposition of reduced silver ions on the gold colloid) . This change can be used to judge an error in assay, as will be described later. As the average particle diameter of metal colloid, about 1 to 500 nm is desirable, and 1 to 100 nm is more desirable.

(Specific Binding Substance)

The specific binding substance is not particularly limited as long as it has an affinity for an analyte. For example, when the analyte is an antigen, an antibody to the antigen may be used. When the analyte is protein, metal ions, or low molecular weight organic compound, aptamers to them may be used. When the analyte is a nucleic acid, such as DNA and RNA, nucleic acid molecules, such as DNA, and RNA, that have complementary sequence to the nucleic acid may be used. When the analyte is avidin, biotin may be used. When the analyte is a specific peptide, a complex specifically binding to the peptide, or the like may be used. In the aforementioned examples, the specific binding substance and the analyte that are related to each other may be switchable therebetween. For example, when the analyte is an antibody, an antigen to the antibody may be used as a specific binding substance. Further, a compound a part of which contains a substance having an affinity for the analyte, as described above, or the like may be used as a specific binding substance.

Specifically, as the antibody, an antiserum prepared from an animal serum immunized with the analyte, an immunoglobulin fraction purified from the antiserum, a monoclonal antibody obtained by cell fusion using animal splenocytes immunized with the analyte, or fragments thereof [for example, F(ab')2, Fab, Fab', or Fv] may by used. Preparation of these antibodies may be performed by using common methods.

(Insoluble Carrier)

It is desirable that the material of the insoluble carrier 21 is porous. For example, a nitrocellulose membrane, a cellulose membrane, an acetyl cellulose membrane, a polysulfone membrane, a polyether sulfone membrane, a nylon membrane, glass fibers, a nonwoven fabric, a fabric, a thread, and the like are desirable.

An assay line is formed on a chromatographic carrier by immobilizing a specific binding substance to an analyte onto a chromatographic carrier. Further, a control site is prepared, if desirable. The specific binding substance may be directly immobilized on a part of the chromatographic carrier by physical or chemical bond. Alternatively, the specific binding substance may be bound to a particle, such as a latex particle, and the particle may be trapped on a part of the chromatographic carrier to immobilize the specific binding substance.

(Sensitizing Solution)

The sensitizing solution can amplify signals by forming a compound that shows color or produces luminescence or the like by reaction of a chemical contained in the solution by a catalystic action of a labeling substance or an analyte. For example, the sensitizing solution is a silver ion solution that makes metal silver precipitate on the metal label by physical development. Particularly, a so-called developer as described in general books in the field of photographic chemistry (for example, "Revised Basic Photographic Engineering - Silver Salt Photography" (The Society of Photography and Imaging of Japan, CORONA PUBLISHING CO., LTD.), "Chemistry of Photography" (Akira Sasai, Photography Industry Publishing Co., Ltd.), "Newest Formulation Handbook" (Shin-ichi Kikuchi, et al, AMIKO Publishing Co., Lid.)) may be used. For example, when a physical developer containing a silver ion containing compound is used as the sensitizing solution, a reducing agent of silver ions can reduce silver ions in the solution to make silver deposit on metal colloid or the like that forms a core for development, as a center.

As another example, an enzyme reaction may be used. For example, a solution of a phenylenediamine compound and a naphthol compound, which forms a dye through an action between a peroxidase label and hydrogen peroxide, may be used. Alternatively, a color-developing substrate for detecting horseradish peroxidase, as disclosed in Non-Patent Document "Clinical Test", Vol. 41, No. 9, p. 1020, "Dyeing Using H2O2-POD System", or the like may be used. Further, a color-developing substrate disclosed in Japanese Unexamined Patent Publication No. 2009-156612 is especially preferable. Further, a system using a metal catalyst, such as platinum particles, instead of an enzyme may be used.

Another example using an enzyme is a system that develops color by using alkaline phosphatase as a label, and 5-bromo-4-chloro-3-indolyl-phosphate disodium salt (BCIP) as a substrate. So far, color development reactions have been described as representative examples. However, any combination of an enzyme and a substrate generally used in enzyme immunoassay may be used. The substrate may be a chemiluminoscent substrate or a fluorescent substrate.

(Silver Ion Containing Compound)

As a silver ion containing compound, an organic silver salt, an inorganic silver salt, or a silver complex may be used. It is desirable that the silver ion containing compound is highly soluble in a solvent, such as water, and examples of the silver ion containing compound is silver nitrate, silver acetate, silver lactate, silver butyrate and silver thiosulfate. Especially, silver nitrate is desirable. It is desirable that the silver complex has a ligand having water-soluble group, such as hydroxyl group or sulfonic group, and an example of the silver complex is hydroxythioether silver, or the like. The inorganic silver salt or the silver complex may contain 0.001 mol/m$^2$ to 0.2 mol/m$^2$ of silver. Further, it is more desirable that the inorganic silver salt or the silver complex contains 0.01 mol/m$^2$ to 0.05 mol/m$^2$ of silver.

(Silver Ion Reducing Agent)

A silver ion reducing agent may be any inorganic or organic material or a mixture thereof as long as the agent can reduce silver ions into silver.

As the inorganic reducing agent, reducing metal salts and reducing metal complex salts having a metal ion with a variable valence, such as $Fe^{2+}$, $V^{2+}$ or $Ti^{3+}$, are preferable. When an inorganic reducing agent is used, it is necessary to complex or reduce the oxidized ion to remove the oxidized ion or to make the oxidized ion harmless. For example, in a system using $Fe^{2+}$ as the reducing agent, a complex of $Fe^{3+}$ that is an oxide may be formed by using citric acid or EDTA to make the oxidized ion harmless. In the system of the present invention, it is desirable to use such an inorganic reducing agent, and it is more desirable to use a metal salt of $Fe^{2+}$.

In the aforementioned embodiment, as a method for amplifying the color-developed state, a method for sensitizing a labeling substance by reducing a silver ion containing compound with a reducing agent was used. However, the sensitizing method in the present invention is not limited to such a method. The sensitizing solution may be any solution as long as the solution can amplify a signal by forming a compound that shows color or produces luminescence by reaction of a chemical contained in the solution by a catalytic action of the labeling substance or the analyte. For example, a solution using an enzyme, as described above, may be used.

In the aforementioned embodiments, immunochromatography was described as the assay method. However, the assay method adopted in the present invention is not limited to immunochromatography. It is not necessary that the system uses a so-called immune reaction. For example, the system may capture an analyte using a nucleic acid, such as DNA or RNR, without using an antibody. Further, the system may capture an analyte using a different small molecule, a peptide, a protein, a complex forming substance, or the like, which has an affinity for the analyte.

**Claims**

1. Am immunochromatographic assay method for performing an assay related to a specimen by reacting a reagent held by a carrier and the specimen with each other, and by obtaining an image signal by imaging a reagent portion that has been reacted, and by measuring, based on the image signal, the color-developed state of the reagent caused by the reaction,

   the method comprising the steps of:

   applying a morphological opening operation to the image signal by using a structuring element the size of which is less than or equal to a signal change width of the reagent portion that has developed color;
   applying a morphological opening operation to the image signal by using a structuring element the size of which is larger than the signal change width of the reagent portion that has developed color;
   obtaining a difference signal by performing a subtraction between the image signals to which the opening operations have been applied respectively; and
   measuring the color-developed state based on the difference signal.

2. An immunochromatographic assay method for performing an assay related to a specimen by reacting a reagent held by a carrier and the specimen with each other, and by obtaining an image signal by imaging a reagent portion that has been reacted, and by measuring, based on the image signal, the color-developed state of the reagent by the reaction,

   the method comprising the steps of:

   applying a level inversion operation to the image signal;
   applying a morphological closing operation to the image signal after the level inversion operation by using a structuring element the size of which is less than or equal to a signal change width of the reagent portion that has developed color;
   applying a morphological closing operation to the image signal after the level inversion operation by using a structuring element the size of which is larger than the signal change width of the reagent portion that has developed color;
   obtaining a difference signal by performing a subtraction between the image signals to which the closing operations have been applied respectively; and
   measuring the color-developed state based on the difference signal.

3. An immunochromatographic assay method, as defined in Claim 1 or 2, wherein the reagent held by the carrier has a band shape with a predetermined width.

4. An immunochromatographic assay method, as defined in any one of Claims 1 to 3, the shape of the structuring element the size of which is less than or equal to the signal change width is more rectangular as the intensity of the image signal is higher, and more arc-shaped as the intensity of the image signal is lower.

5. An immunochromatographic assay method, as defined in any one of Claims 1 to 4, wherein the shape of the structuring element the size of which is less than or equal to the signal change width is bilaterally asymmetrical.

6. An immunochromatographic assay method, as defined in any one of Claims 1 to 5, wherein the size of the structuring element that is less than or equal to the signal change width is 60% or greater of the signal change width.

7. An immunochromatographic assay method, as defined in Claim 6, wherein the size of the structuring element that is less than or equal to the signal change width is 80% or greater of the signal change width.

8. An immunochromatographic assay method, as defined in any one of Claims 1 to 7, wherein the size of the structuring element that is larger than the signal change width is 1.2 times or more as large as the signal change width.

9. An immunochromatographic assay method, as defined in Claim 8, wherein the size of the structuring element that is larger than the signal change width is 1.5 times or more as large as the signal change width.

10. An immunochromatographic assay apparatus comprising:

   a loading means that loads a carrier holding a reagent;
   an imaging means that obtains an image signal by imaging a reagent portion that has been reacted with a specimen; and
   a measuring means that measures, based on the image signal, the color-developed state of the reagent by the reaction,
   the apparatus comprising:

      a morphological operation unit that applies a morphological opening operation to the image signal by using a structuring element the size of which is less than or equal to a signal change width of the reagent portion that has developed color, and that applies a morphological opening operation to the image signal by using a structuring element the size of which is larger than the signal change width of the reagent portion that has developed color;
      a subtraction processing unit that obtains a difference signal by performing a subtraction between the image signals to which the opening operations have been applied respectively; and
      a measuring means that measures the color-developed state based on the difference signal.

11. An immunochromatographic assay apparatus comprising:

   a loading means that loads a carrier holding a reagent;
   an imaging means that obtains an image signal by imaging a reagent portion that has been reacted with a specimen; and
   a measuring means that measures, based on the image signal, the color-developed state of the reagent by the reaction,
   the apparatus comprising:

      a means for applying a level inversion operation to the image signal;
      a morphological operation unit that applies a morphological closing operation to the image signal by using a structuring element the size of which is less than or equal to a signal change width of the reagent portion that has developed color, and that applies a morphological closing operation to the image signal by using a structuring element the size of which is larger than the signal change width of the reagent portion that has developed color;
      a subtraction processing unit that obtains a difference signal by performing a subtraction between the image signals to which the closing operations have been applied respectively; and
      a measuring means that measures the color-developed state based on the difference signal.

# FIG.1

# FIG.2

EP 2 623 980 A1

# FIG.3

| | |
|---|---|
| 100V~240V → | ELECTRIC POWER SOURCE UNIT 100V→12V  *100* |

DISPLAY UNIT  *11*

PRESSURE MECHANISM  *30,34*

12V

IMAGE PROCESSING UNIT  *81*

*102* BATTERY

SWITCH UNIT  *101*

CONTROL UNIT  *80*

CAMERA  *42*

BATTERY CAPACITY MONITOR UNIT  *103*

OPERATION UNIT  *12*

CAMERA  *52*

# FIG.4

*30*  *31*  *34*

*35*

*31a*

*32*  *36*

*33*

*27*  *28*  *20*

*72*  *73*

*39*  *38*

*21*  *22*

**FIG.5**

**FIG.6**

**FIG.7**

# FIG.8

(A)

f (x)

●—●MASK SIZE

AFTER DILATION PROCESSING

BEFORE DILATION PROCESSING

X

(B)

f (x)

●—●MASK SIZE

BEFORE EROSION PROCESSING

AFTER EROSION PROCESSING

X

(C)

f (x)

CONVEX SIGNAL
CHANGE PART
SMALLER THAN MASK SIZE

●—●MASK SIZE

BEFORE OPENING OPERATION

AFTER OPENING OPERATION

X

(D)

f (x)

●—●MASK SIZE

AFTER CLOSING OPERATION

BEFORE CLOSING OPERATION

CONCAVE SIGNAL
CHANGE PART SMALLER THAN MASK SIZE

X

# FIG.9

S1

```
RECEIVE IMAGED IMAGE
```

S2

```
APPLY AN OPENING
OPERATION FOR
DETECTING A SIGNAL
```

S3

```
APPLY AN OPENING
OPERATION FOR
GENERATING A STANDARD
FOR DETECTING A SIGNAL
```

S4

```
GENERATE AN IMAGE
SIGNAL FOR DETECTION
```

S5

```
DISPLAY ASSAY RESULT
```

# FIG.10

(A)

SIGNAL LEVEL

POSITION

(B)

SIGNAL LEVEL

D

a

b

POSITION

(C)

SIGNAL LEVEL

b

POSITION

(D)

SIGNAL LEVEL

a

POSITION

## INTERNATIONAL SEARCH REPORT

| | International application No. |
|---|---|
| | PCT/JP2011/005440 |

A.   CLASSIFICATION OF SUBJECT MATTER
*G01N33/543(2006.01)i, G01N21/78(2006.01)i*

According to International Patent Classification (IPC) or to both national classification and IPC

B.   FIELDS SEARCHED

Minimum documentation searched (classification system followed by classification symbols)
G01N33/543, G01N21/78

Documentation searched other than minimum documentation to the extent that such documents are included in the fields searched
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| A | JP 2009-150856 A  (Panasonic Corp.),<br>09 July 2009 (09.07.2009),<br>paragraph [0041]<br>(Family: none) | 1-11 |
| A | JP 2009-98080 A  (Panasonic Corp.),<br>07 May 2009 (07.05.2009),<br>entire text; all drawings<br>(Family: none) | 1-11 |
| A | JP 2006-507486 A  (Qinetiq Ltd.),<br>02 March 2006 (02.03.2006),<br>claim 2<br>& US 2006/0036369 A1      & GB 224626 D<br>& EP 1554680 A          & WO 2004/038633 A1<br>& DE 60310120 D          & AT 347150 T<br>& AU 2003274337 A | 1-11 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 December, 2011 (13.12.11) | 20 December, 2011 (20.12.11) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
| --- |
| PCT/JP2011/005440 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2004-502129 A  (Sir Mortimer B., Davis Jewish General Hospital), 22 January 2004 (22.01.2004), page 17 & US 6511849 B1          & EP 1230383 A & WO 2000/060126 A2      & CA 2268695 A & AU 4224400 A            & IL 145826 D | 1-11 |
| A | JP 2005-345310 A  (Sadahiko NAGAE), 15 December 2005 (15.12.2005), paragraph [0026] (Family: none) | 1-11 |
| A | JP 2009-513939 A  (Medion Diagnostics GmbH), 02 April 2009 (02.04.2009), entire text; all drawings & US 2007/0248983 A1     & EP 1646876 A & WO 2005/005991 A1     & DE 10330982 A & CA 2531688 A          & KR 10-2006-0059958 A & CN 1816746 A          & EG 24046 A & NZ 544545 A           & ZA 200601129 A & BRA PI0411866         & RU 2353293 C & AU 2004256210 A       & IL 172998 D & HK 1094721 A          & AT 514092 T | 1-11 |
| A | WO 03/100425 A1  (Jokoh Co., Ltd.), 04 December 2003 (04.12.2003), entire text; all drawings & AU 2003242389 A | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008139297 A
- JP 2009133813 A
- JP 2009287952 A
- JP 8272961 A
- JP 9248291 A
- JP 9091421 A
- JP 2009156612 A

**Non-patent literature cited in the description**

- Clinical Test. *Dyeing Using H2O2-POD System,* vol. 41 (9), 1020